# EUROPEAN PATENT SPECIFICATION

(11) **EP 4 316 360 B1**
(45) Date of publication and mention of the grant of the patent: **09.07.2025**
(21) Application number: 23189638.2
(22) Date of filing: 04.08.2023
(51) Int. Cl.: A61B 5/00, A61B 5/103, A61B 5/11, A61B 5/107

(54) **SPINE MEASURING DEVICE, SPINE MEASURING SYSTEM AND MEASURING METHOD**
WIRBELSÄULENMESSVORRICHTUNG, WIRBELSÄULENMESSSYSTEM UND MESSVERFAHREN
DISPOSITIF DE MESURE DE COLONNE VERTÉBRALE, SYSTÈME DE MESURE DE COLONNE VERTÉBRALE ET PROCÉDÉ DE MESURE

(30) Priority: 05.08.2022 CN 202222058140 U
(43) Date of publication of application: 07.02.2024
(73) Proprietor: Forethought (Shanghai) Medical Technology Co., Ltd., 201203 Shanghai (CN)
(72) Inventor: He, Dan, Shanghai, 201203 (CN)
(74) Representative: Vesterinen, Jussi Tapio

(56) References cited:
- CN-A- 113 786 188
- CN-U- 215 227 638
- DE-A1- 4 336 144
- KR-A- 20150 059 244
- US-A1- 2006 015 042
- US-B1- 6 258 047
- MANNION ANNE F. ET AL: "A new skin-surface device for measuring the curvature and global and segmental ranges of motion of the spine: reliability of measurements and comparison with data reviewed from the literature", EUROPEAN SPINE JOURNAL, vol. 13, no. 2, 6 December 2003 (2003-12-06), Berlin/Heidelberg, pages 122 - 136, XP093125429, ISSN: 0940-6719, Retrieved from the Internet <URL:https://www.ncbi.nlm.nih.gov/pmc/articles/PMC3476568/pdf/586_2003_Article_618.pdf> DOI: 10.1007/s00586-003-0618-8

## Description

### TECHNICAL FIELD

The present disclosure relates to the technical field of detection instruments, in particular to a spine measuring device, a spine measuring system, a measuring method and a control device.

### BACKGROUND

Scoliosis detection, as an important item of orthopedic detection, is of great significance in the prevention, correction and diagnosis and treatment of adolescent spinal diseases.

At present, the conventional detection methods comprise a scoliosis measuring ruler, an X-ray, a CT and other image detection methods. In recent years, large-scale three-dimensional optical detection devices have been developed. The scoliosis measuring ruler has a simple structure, but it is complicated for doctors to operate, and the result is not intuitive. Fluoroscopic image inspection has a large amount of radiation, which is potentially harmful to human body, and the inspection cost is high. However, large-scale three-dimensional optical devices are expensive.

Therefore, it is necessary to design a spine measuring device which is simple in structure and convenient use to solve the above problems.

CN113786188A discloses a scoliosis detection device and a scoliosis detection method. The scoliosis detection device comprises a handheld shell, a motion sensor, a geomagnetic sensor, a rechargeable battery, a control panel and a target generator, wherein the motion sensor, the geomagnetic sensor, the rechargeable battery and the control panel are mounted in the handheld shell, and the target generator is mounted in the bottom middle of the handheld shell. A pair of rollers is positioned on each side of the target generator.

### SUMMARY

The invention is defined by the appended claims.

The present disclosure aims to provide a spine measuring device, a spine measuring system, a measuring method and a control device. A traveling wheel moves on the back of a human body, a measuring wheel moves along the spine, and each sensor acquires data, so as to obtain scoliosis data. The device is simple in structure and convenient to use. Many technical effects that can be produced by the preferred technical scheme among the technical schemes provided by the present disclosure are described in detail hereinafter.

In order to achieve the above objective, the present disclosure provides the following technical scheme.

The present disclosure provides a spine measuring device as recited in claim 1.

Further, the spine measuring device further comprises a pressure sensor configured to measure the pressure on the measuring wheel, the pressure sensor is connected with the control device, and the pressure sensor is provided between the mounting frame and the elastic element.

Further, the measuring wheel is provided on a central axis of the measuring body; the first detection device comprises a plurality of first sensors, and the plurality of first sensors are arranged along a rolling direction of a wheel surface of the measuring wheel.

Further, a radial section of any position of the measuring wheel is circular, a diameter of the middle position of the measuring wheel is larger than diameters of the left and right positions of the measuring wheel; the plurality of first sensors are arranged in three groups along the rolling direction of the wheel surface of the measuring wheel, one group of the first sensors is provided at the middle position of the measuring wheel, and the other two groups of the first sensors are provided at both sides of the measuring wheel.

Further, an arc angle between sensing points of two adjacent of the first sensors and the center of the measuring wheel is less than 72 degrees; alternatively, there are not less than 20 first sensors in each circle.

Further, an optical sensor and a light emitting device are provided on the mounting frame supporting the measuring wheel, the optical sensor and the light emitting device are provided above the measuring wheel, an outer surface of an outer wheel of the measuring wheel is provided with reflective strips with different light reflectivity at intervals along the rolling direction, and the intensity of the reflected light is received by the optical sensor for judging a rolling speed and a travel distance of the measuring wheel.

Further, the measuring wheel comprises an inner wheel and an outer wheel, the outer wheel is sleeved on the inner wheel, the inner wheel is fixed, and the outer wheel rolls with respect to the inner wheel; more than two fourth sensors are provided in a downward area of the outer surface of the inner wheel, the fourth sensors are provided along a circumferential direction of the inner wheel, the fourth sensors are connected with the control device, and the fourth sensors are configured to test a pressure change between the measuring wheel and skin.

Further, the balance wheel is further provided with a third sensor, the third sensor is provided on the wheel surface or a wheel shaft of the balance wheel, and the third sensor is configured to detect the position where the wheel surface of the balance wheel is in contact with a measured person.

Further, either of a pair of balance wheels provided oppositely is provided with a second sensor, the second sensor is connected with the control device, and the second sensor is configured to detect a moving distance of the balance wheels.

Further, a distance between the pair of balance wheels provided oppositely is greater than a width of the measuring wheel; at least four balance wheels rotate independently, and each of the balance wheels has a cylindrical structure.

The present disclosure provides a spine measurement system, comprising an external host and the spine measuring device, wherein the external host is in communication connection with the spine measuring device.

The present disclosure provides a measuring method for detecting a spine by using the spine measuring device, comprising:
acquiring pressure data of each coordinate position of the spine; and identifying a position of a seventh cervical spinous process based on the pressure data for positioning the detection of cervical vertebra.

Further, identifying the position of the seventh cervical spinous process based on the pressure data specifically comprises: acquiring pressure change data based on the pressure data of each coordinate position; searching for target pressure change data which conforms to a preset seventh cervical pressure abrupt change range according to the pressure change data; determining a corresponding measurement coordinate position according to the target pressure change data; identifying the position of the seventh cervical spinous process based on the corresponding measurement coordinate position.

The preferred technical scheme of the present disclosure can at least produce the following technical effect.

According to the spine measuring device provided by the present disclosure, when the measuring wheel rolls along skin, the first detection device can detect the movement of the measuring wheel and calculate a movement speed and a rolling distance of the measuring wheel. Based on the current movement speed, the rolling distance and each coordinate position acquired by the spatial coordinate acquisition device, spatial curve data is acquired and generated, so as to obtain scoliosis data. The device is simple in structure and is convenient to use.

The present disclosure provides a measuring method, which acquires pressure change data through a pressure sensor of a spine measuring device, accurately identifies and positions the seventh cervical vertebra, and finds the starting point of measurement accurately. Data acquisition also starts from the seventh cervical vertebra, thus ensuring the accuracy of subsequent data acquisition and analysis.

### BRIEF DESCRIPTION OF THE DRAWINGS

In order to explain the embodiments of the present disclosure or the technical schemes in the prior art more clearly, the drawings that need to be used in the description of the embodiments or the prior art will be briefly introduced hereinafter. Obviously, the drawings in the following description are only some embodiments of the present disclosure. For those skilled in the art, other drawings can be obtained according to these drawings without any creative effort.
FIG. 1 is a schematic structural diagram of a spine measuring device according to Embodiment 1 of the present disclosure.
FIG. 2 is a schematic diagram of the partial structure of the using state of a spine measuring device according to Embodiment 1 of the present disclosure.
FIG. 3 is a schematic diagram of the partial structure of a spine measuring device according to Embodiment 1 of the present disclosure.
FIG. 4 is a schematic diagram of the installation structure of a measuring wheel according to Embodiment 1 of the present disclosure.
FIG. 5 is a schematic diagram of the partial structure of the using state of a spine measuring device according to Embodiment 2 of the present disclosure.
FIG. 6 is a schematic structural diagram of a spine measuring device according to Embodiment 2 of the present disclosure.
FIG. 7 is a schematic structural diagram of a mounting frame and a measuring wheel according to Embodiment 2 of the present disclosure.
FIG. 8 is a schematic structural diagram of a measuring wheel according to Embodiment 2 of the present disclosure.
FIG. 9 is a schematic structural diagram of an inner wheel of a measuring wheel according to Embodiment 2 of the present disclosure.
FIG. 10 is a schematic structural diagram of an inner wheel of a measuring wheel according to Embodiment 2 of the present disclosure.
FIG. 11 is a schematic structural diagram of a mounting frame according to Embodiment 2 of the present disclosure.
FIG. 12 is a cross-sectional schematic diagram of a mounting frame according to Embodiment 2 of the present disclosure.
FIG. 13 is a flowchart schematic diagram of an embodiment of a spine measuring method according to the present disclosure.

In the figures, 1. Measuring body; 101. Handle; 2. Measuring wheel; 201. Inner wheel; 2011. Arc strip; 202. Outer wheel; 3. Balance wheel; 4. First sensor; 401. Intermediate sensor; 402. Left sensor; 403. Right sensor; 5. Simulated measured person; 501. Spine; 502. High skin point; 6. Pressure sensor; 7. Angle detection device; 8. Mounting frame; 9. Elastic element; 10. Third sensor; 11. Second sensor; 12. Fourth sensor; 13. Optical sensor; 14. Light emitting device; 15. Reflective strip; 1501. High-reflectivity strip; 1502. Low-reflectivity strip; 16. Detection window.

### DETAILED DESCRIPTION OF THE EMBODIMENTS

In order to make the object, technical scheme and advantages of the present disclosure clearer, the technical scheme of the present disclosure will be described in detail hereinafter. Obviously, the described embodiments are only part of the embodiments of the present disclosure, rather than all of the embodiments. The invention is defined by the appended claims.

The present disclosure provides a spine measuring device, comprising a measuring body 1, a measuring wheel 2, a balance wheel 3, a first detection device, a spatial coordinate acquisition device 7 and a control device, wherein the measuring wheel 2 is provided at one end of the measuring body 1, at least four balance wheels 3 are symmetrically provided in rows at both sides of the measuring wheel 2 and are all connected with the measuring body 1, the first detection device and the spatial coordinate acquisition device 7 are both connected with the control device, the first detection device is configured to detect the movement of the measuring wheel 2, and the spatial coordinate acquisition device 7 is configured to detect a spatial angle value.

When the measuring wheel 2 rolls along skin, the first detection device can detect the movement of the measuring wheel 2 and calculate a movement speed and a rolling distance of the measuring wheel 2. Based on the current movement speed, the rolling distance and each coordinate position acquired by the spatial coordinate acquisition device 7, spatial curve data is acquired and generated, so as to obtain scoliosis data. The device is simple in structure and is convenient to use.

Next, the present disclosure provides preferred embodiments of both spine measuring devices.

### Embodiment 1:

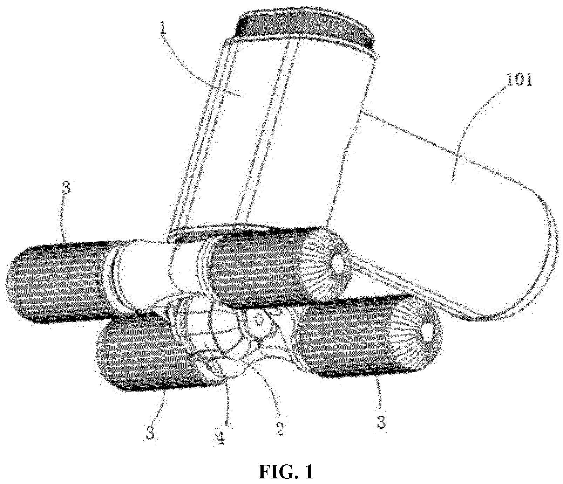

Referring to FIGS. 1 to 4 of the specification, the present disclosure provides a spine measuring device, comprising a measuring body 1, a measuring wheel 2, a balance wheel 3, and a control device. The measuring wheel 2 is provided at one end of the measuring body 1. A plurality of first sensors 4 are provided on the measuring wheel 2, and the first sensors 4 are configured to detect the position where the measuring wheel 2 is located. At least four balance wheels 3 are provided at one end of the measuring body 1 close to the measuring wheel 2. At least four balance wheels 3 are symmetrically provided in two rows at both sides of the measuring wheel 2. The control device is provided in the measuring body 1. The control device is connected with the plurality of first sensors 4 and the spatial coordinate acquisition device 7, respectively.

The spatial coordinate acquisition device 7 comprises a triaxial angle sensor. The triaxial angle sensor is provided on the measuring body 1 and is fixed with respect to the position of the measuring wheel 2. The triaxial angle sensor can also be an acceleration sensor or a gyroscope sensor or be replaced by other MEMS motion sensors. When the triaxial angle sensor is combined with a geomagnetic sensor, the rotation angle change of the spatial X, Y and Z axes of the positions where the triaxial angle sensor and the geomagnetic sensor are located can be obtained. The triaxial angle sensor is installed inside the measuring body 1. Four balance wheels 3 are in contact with four skin high points 502 of the skin. The measuring wheel 2 always moves along the central position of the spine 501. With the movement of the measuring body 1, the spatial angle value of each test point can be obtained, and the spatial angle change comprises a pitch angle, a roll angle and a heading angle.

The spatial curves can be calculated using the method of eliminating multiple types of error data; a first type is that the contact point between the test wheel and the skin is a real test point, but the position of the triaxial angle sensor is fixed with respect to the test wheel, so that it is necessary to eliminate the error of the relative distance. After correction, the position of the triaxial angle sensor coincides with the contact point between the test wheel and the skin, which eliminates the sagittal data error.

Referring to FIGS. 1 to 4 of the specification, the measuring body 1 has a long strip structure. One side of the measuring body 1 is provided with a handle 101, and the measuring wheel 2 is provided on the central axis of the measuring body 1.

The first detection device comprises a plurality of first sensors 4. The plurality of first sensors 4 are arranged along the rolling direction of the wheel surface of the measuring wheel 2. When the measuring wheel 2 rolls along the skin, the sensors are in contact with the skin in sequence. The current movement speed and rolling distance can be calculated by the contact interval time and the arc length of the sensor.

The plurality of first sensors 4 are arranged in three groups along the rolling direction of the wheel surface of the measuring wheel 2, namely, the first sensor in the middle position (referred to as the middle sensor 401), the first sensor in the left position (referred to as the left sensor 402) and the first sensor in the right position (the right sensor 403). The first sensor 4 can be a capacitance sensor. For example, the first sensor 4 is a multi-channel surface capacitance sensor. After the multi-channel surface capacitance sensor is in contact with the skin, the capacitance changes, and the area of the skin in contact with the surface of the measuring wheel 2 can be calculated by calculating the capacitance change.

The radial section of any position of the measuring wheel 2 is circular, and the diameter of the middle position of the measuring wheel 2 is larger than the diameters of the left and right positions of the measuring wheel 2. The middle sensor 401 is provided at the middle position of the measuring wheel 2. The left sensor 402 is provided at the left position of the measuring wheel 2, and the right sensor 403 is provided at the right position of the measuring wheel 2. Since the shape of the body surface area where the spine is located is convex in the neck area, only the middle sensor 401 can be in contact with the skin, while the left sensor 402 and the right sensor 403 cannot be in contact with the skin. The body surface shape of the thoracic vertebra and lumbar vertebra gradually transits from an upper convex part to a lower concave part. At this time, the contact area between three groups of sensors and the skin gradually increases, in which at the beginning, only the middle sensor 401 is in contact with the skin, and then the left sensor 402 and the right sensor 403 are in contact with the skin, until the area of the middle sensor 401 in contact with a very concave area decreases or even is not in contact with the very concave area. In this way, it can be judged that the spine measuring device is located in the measured area of the human body.

The upper three groups of capacitance sensors can also be used to evaluate the accuracy of the operation method of the measured person. When the three groups of sensors perform measurement in normal state, the data of the left sensor 402 and the right sensor 403 are basically symmetrical because of the symmetry of the human body. If serious asymmetry occurs, there are two situations: the first situation is an operation error since the measuring body 1 is skewed left and right or the measuring process deviates from the center line; the second situation is that the measured person has serious local scoliosis deformation. It can be determined whether it is caused by the test operation error or severe scoliosis by judging the synchronization of the symmetry between the measured scoliosis data and the three groups of capacitance sensor data, so as to evaluate the reliability and accuracy of the test operation.

Further, the sensing points of each group of first sensors 4 are uniformly distributed on the wheel surface of the measuring wheel 2 to form three circles of sensing points of the sensors. In each circle of sensing points of the sensors, an arc angle between sensing points of two adjacent of the first sensors 4 and the center of the measuring wheel 2 is less than 72 degrees; alternatively, there are not less than 20 first sensors 4 in each circle.

Referring to FIGS. 1 to 4 of the specification, the spine measuring device further comprises a mounting frame 8 and an elastic element 9. The mounting frame 8 is provided at one end of the measuring body 1. The measuring wheel 2 is installed on the mounting frame 8 through a rotating shaft so that the measuring wheel 2 can rotate with respect to the mounting frame 8. The elastic element 9 is provided in the measuring body 1. One end of the elastic element 9 abuts against the measuring body 1, and the other end of the elastic element 9 is connected with the mounting frame 8, so that the measuring wheel 2 can move in the axial direction of the elastic element 9. The elastic element 9 can be an elastic column or a telescopic spring. The wheel surface of the measuring wheel 2 can always be attached to the skin surface by providing the elastic element 9. The measuring wheel 2 is located in the middle of the balance wheels 3. The width of the measuring wheel 2 is smaller than the gap width between each pair of balance wheels 3. When the human spine is convex in the body surface area, the wheel surface of the measuring wheel 2 can be shrunk above the tangent plane of the wheel surface of the four balance wheels 3, and the protruding part of the human body is just located in the gap between each pair of balance wheels 3, so that the balance wheels 3 can continue to be in contact with the skin surface.

Further, the spine measuring device further comprises a pressure sensor 6. The pressure sensor 6 is sandwiched between the mounting frame 8 and the elastic element 9 and is configured to measure the pressure on the measuring wheel 2. The pressure sensor 6 is configured to measure the pressure change, in which when the measuring wheel 2 moves on the uneven skin surface, the measuring wheel 2 will fluctuate with the unevenness, and the elastic element 9 will compress accordingly. Because the human body will be particularly convex in the seventh cervical spinous process, the pressure may abruptly change. By recording the abruptly changing pressure signal, the measurement position of the seventh cervical spinous process can be identified, which is used to further optimize the position of pathological points after measurement.

Referring to FIGS. 1 to 4 of the specification, the number of balance wheels 3 can be set as four, six, eight, ten, etc. Preferably, four balance wheels 3 are provided. The four balance wheels 3 rotate independently, and each balance wheel 3 has a cylindrical structure. The four balance wheels 3 are provided at one end of the measuring body 1 close to the measuring wheel 2. The four balance wheels 3 are symmetrically provided at both sides of the measuring wheel 2 in two rows, wherein a pair of balance wheels 3 which are oppositely provided (namely, two balance wheels 3 on the left and right sides of the measuring wheel 2) are respectively provided with second sensors 11, and the second sensors 11 are configured to detect the moving distance of the balance wheels 3. The second sensor 11 can be an angle measuring sensor, which independently measures the relative moving distance between the two wheel surfaces and the skin, calculates the moving distance of the central axis position of the measuring body 1 through the moving distance between the left and right wheels and the interval between the left and right wheels and the skin, and compares it with the data measured by the balance wheel 3 itself.

Further, the balance wheel 3 is further provided with a plurality of third sensors 10. The plurality of third sensors are arranged on the wheel surface of the balance wheel 3 along the same axis direction. The third sensors 10 are configured to detect the position where the wheel surface of the balance wheel 3 is in contact with the measured person. The four balance wheels 3 are all cylindrical wheel surfaces, and the position where the wheel surface is in contact with the skin needs to be confirmed. A group of third sensors 10 is added to the wheel surface, and the third sensors 10 can be pressure sensors or capacitance sensors. The contact center position can be determined by judging the extreme value of pressure intensity or the extreme value of capacitance of this group of sensors in contact with the skin, so as to confirm the position where the wheel surface is in contact with the skin. The accuracy of the test operation can also be evaluated by judging the contact between the wheel surface and the skin. During normal measurement, none of the four balance wheels 3 are allowed to leave the skin, and the contact points between the left and right wheel surfaces of the two groups of balance wheels 3 and the skin should be symmetrical. Through these evaluations, the accuracy of the test operation can be improved.

In this embodiment, when the measuring wheel 2 rolls along the skin, the first sensors 4 are in contact with the skin in sequence. The current movement speed and rolling distance can be calculated by the contact interval time and the arc length of the first sensor. The second sensor 11 detects the moving distance of the balance wheel 3, and calculates the moving distance of the measuring wheel 2 by calculating the moving distance of the left and right wheels and the interval between the left and right wheels and the skin when the balance wheel 3 travels along a curved path. The shape of the body surface area where the spine is located is convex in the neck area, and the body surface shape of the thoracic vertebra and lumbar vertebra gradually transits from an upper convex part to a lower concave part. The first sensor 4 can detect which measured area of the human body the measuring wheel 2 is located in. When the triaxial angle sensor and the geomagnetic sensor are used to measure the spatial angle value of each test point, in combination with the movement data of the measuring wheel 2, a series of spatial curves of test points can be calculated, and the angle change value of spinal torsion can be calculated, so as to obtain the scoliosis data. The device is simple in structure and convenient to use. As the control unit of the spine measuring device, the control device can be provided with a variety of circuits, and the types and numbers of the circuits can be arranged according to the actual needs. The control device has calculation function, and can calculate the data acquired by each sensor to obtain scoliosis data.

### Embodiment 2:

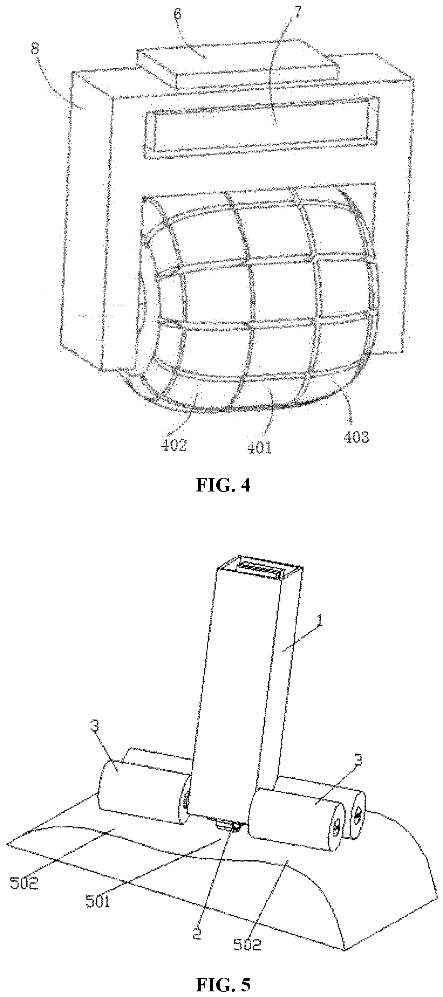

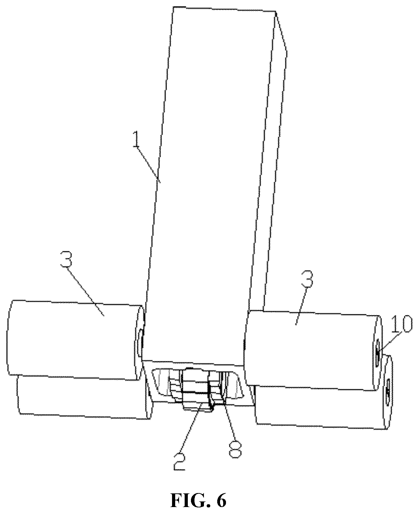

Referring to FIGS. 5 to 12 of the specification, a spine measuring device comprises a measuring body 1, a measuring wheel 2, a balance wheel 3, a first detection device, a spatial coordinate acquisition device 7 and a control device, wherein the measuring wheel 2 is provided at one end of the measuring body 1. At least four balance wheels 3 are symmetrically provided in rows at both sides of the measuring wheel 2 and are all connected with the measuring body 1. The first detection device and the spatial coordinate acquisition device 7 are both connected with the control device. The first detection device is configured to detect the movement of the measuring wheel 2, and the spatial coordinate acquisition device 7 is configured to detect a spatial angle value.

The spatial coordinate acquisition device 7 is provided on the mounting frame. The spatial coordinate acquisition device 7 is opposite to the position of the measuring wheel 2. The spatial coordinate acquisition device 7 comprises a 3-axis accelerometer, a 3-axis gyroscope and a 3-axis magnetometer, forming a group of 9-axis sensing devices, which can measure the angle changes of the spatial pitch angle, rolling angle and heading angle and their absolute values with respect to the magnetic field of the earth during the movement of the measuring wheel 2 in real time. The spatial coordinate acquisition device 7 is installed inside the measuring body 1. Four balance wheels 3 are in contact with four skin high points 502 of the skin, and the measuring wheel 2 always moves along the central position of the spine 501. With the movement of the measuring body 1, the spatial angle value of each test point can be obtained.

The spatial curves can be calculated using the method of eliminating multiple types of error data; a first type is that the contact point between the test wheel and the skin is a real test point, but the position of the triaxial angle sensor is fixed with respect to the test wheel, so that it is necessary to eliminate the error of the relative distance. After correction, the position of the triaxial angle sensor coincides with the contact point between the test wheel and the skin, which eliminates the sagittal data error.

The spine measuring device further comprises a mounting frame 8 and an elastic element 9. The mounting frame 8 is provided at one end of the measuring body 1. The measuring wheel 2 is installed on the mounting frame 8. The elastic element 9 is provided in the measuring body 1. One end of the elastic element 9 abuts against the measuring body 1, and the other end thereof is connected with the mounting frame 8. The elastic element 9 is provided in the measuring body 1. One end of the elastic element 9 abuts against the measuring body 1, and the other end of the elastic element 9 is connected with the mounting frame 8, so that the measuring wheel 2 can move in the axial direction of the elastic element 9. The elastic element 9 can be an elastic column or a telescopic spring. The wheel surface of the measuring wheel 2 can always be attached to the skin surface by providing the elastic element 9. The measuring wheel 2 is located in the middle of the balance wheels 3. When the human spine is convex in the body surface area, the wheel surface of the measuring wheel 2 can be shrunk above the tangent plane of the wheel surface of the four balance wheels 3, and the protruding part of the human body is just located in the gap between each pair of balance wheels 3, so that the balance wheels 3 can continue to be in contact with the skin surface.

The measuring wheel 2 is provided at the front end of the measuring body 1 through the mounting frame 8. The measuring wheel 2 comprises an inner wheel 201 and an outer wheel 202. The outer wheel 202 is sleeved on the inner wheel 201, the inner wheel 201 is fixed, and the outer wheel 202 can roll with respect to the inner wheel 201. More than two fourth sensors 12 are provided in the downward area of the outer surface of the inner wheel 201. The fourth sensors 12 are provided along the circumferential direction of the inner wheel 201. The fourth sensors 12 are connected with the control device, and the fourth sensors 12 are configured to test the pressure change between the measuring wheel 2 and the skin.

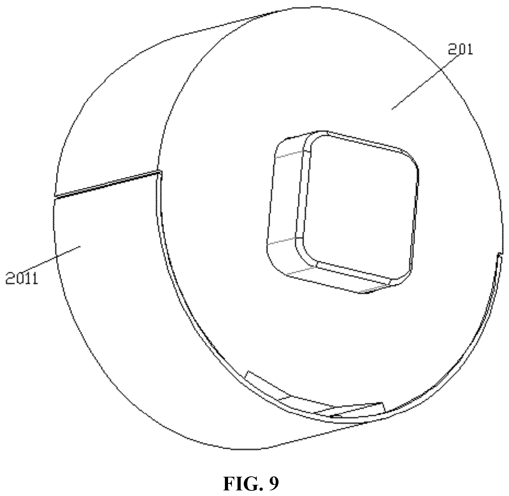

Referring to FIGS. 9-10, the inner wheel 201 is provided with grooves in the direction facing the skin contact surface, and at least three pressure sensors (the fourth sensors 12) are provided, respectively. A plurality of pressure sensors are provided along the circumferential direction of the inner wheel 201. The inner wheel 201 and the outer wheel 202 can slide and wheel with each other. The outer surface of the outer wheel 202 is rough, and the inner surface is smooth. The outer wheel 202 can be made of plastic, Teflon, nylon and other materials. The outer surface of the inner wheel 201 is smooth. Axles are provided at both ends of the inner wheel 201, which are fixed on the mounting frame 8 through the axles.

When the measuring wheel 2 is pressed to the skin surface as a whole for rolling measurement, the outer wheel 202 is in contact with the skin. Because the friction between the outer surface of the outer wheel 202 and the skin is greater than that between the inner surface of the outer wheel 202 and the outer surface of the inner wheel 201, the outer wheel 202 rolls, the inner wheel 201 does not move, and the reaction force of the pressure on the skin is transmitted to the inner wheel 201 through the outer wheel 202. Because the inner wheel 201 is provided with a plurality of pressure sensors at the position opposite to the skin surface, the pressure change between the inner wheel 201 and the skin can be measured. In addition, when a plurality of sensors measure the pressure at the same time, by comparing the pressure outputs of different sensors, the change of the center point applying the pressure on the skin and the surface of the inner wheel 201 can be obtained. When the center point applying the pressure is not the pressure sensor in the middle position set by the system, it can be judged that the measurement operation is deviated.

The pressure change of the skin measured by the pressure sensor on the measuring wheel 2 has the beneficial effect that it can be judged whether the measuring wheel 2 rolls over the skin surface on the spinal ridge skeleton or rolls over the skin surface on the spinal ridge skeleton space by identifying the trend of pressure change (because the skin hardness on the spinal ridge skeleton is high, the skin hardness on the spinal ridge skeleton space is low).

As for the inner wheel 201, the overall circumferential outer contour of the outer wheel 201 has a smooth circumferential curved surface, and the outer wheel 201 comprises a wheel body and an arc-shaped strip 2011. The wheel body is provided with an installation groove matched with the arc-shaped strip 2011, and a groove space in which the fourth sensor 12 is installed is provided on the wheel body.

An optical sensor 13 and a light emitting device 14 are provided on the mounting frame 8 supporting the measuring wheel 2. The optical sensor 13 and the light emitting device 14 are provided above the measuring wheel 2. An outer surface of an outer wheel of the measuring wheel 2 is provided with reflective strips 15 with different light reflectivity at intervals along the rolling direction, and the intensity of the reflected light is received by the optical sensor 13 for judging a rolling speed and a travel distance of the measuring wheel 2.

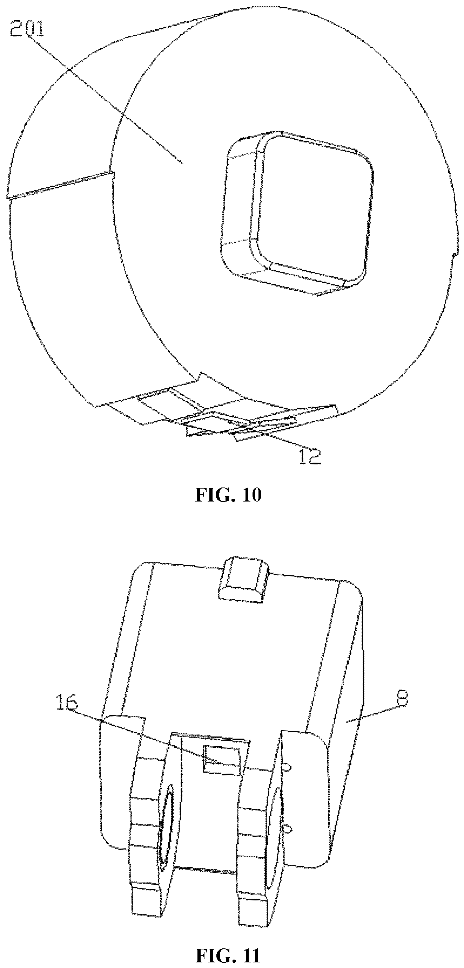

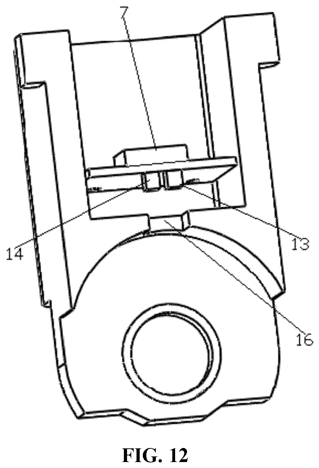

Referring to FIGS. 11 and 12, an optical sensor 13 and a light emitting device 14 are provided on the mounting frame 8 supporting the measuring wheel 2. The outer surface of the outer wheel 202 of the measuring wheel 2 is provided with reflective strips 15 with different light reflectivity at intervals along the rolling direction. Referring to FIG. 11, the mounting frame 8 is provided with a detection window 16, so that the emitted light can be emitted to the surface of the outer wheel 202, and the reflected light can be emitted to the optical sensor 13. When the outer wheel 202 rolls, the reflective strips 15 with different reflectivity provided at intervals are illuminated and reflected in sequence. The optical sensor 13 judges the rolling speed and the travel distance of the outer wheel 202 according to the intensity of the received reflected light.

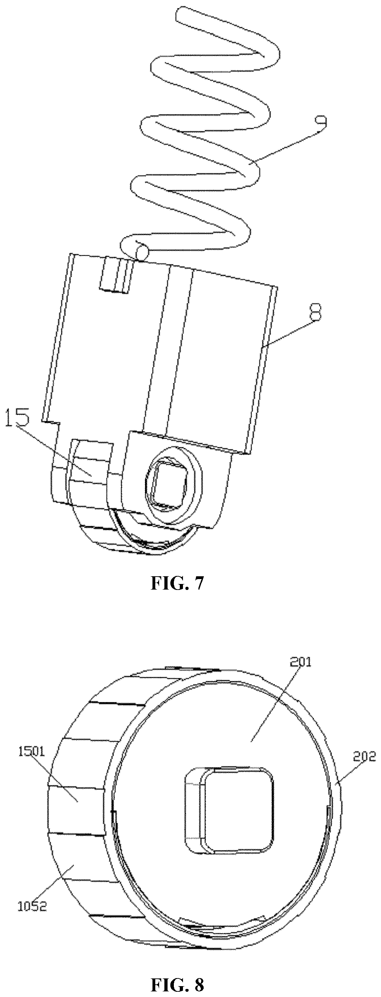

Referring to FIG. 8, the outer wheel 201 is provided with reflective strips 15. The reflective strips 15 are divided into high-reflectivity strips 1501 and low-reflectivity strips 1502. The high-reflectivity strips 1501 and the low-reflectivity strips 1502 are distributed at intervals along the circumferential direction of the outer wheel 201 in sequence.

Referring to FIGS. 11 and 12, the mounting frame 8 comprises a cylindrical part and rotating shaft supporting plates. Two rotating shaft supporting plates are provided at the bottom of the cylindrical part. The two rotating shaft supporting plates are opposite to each other to fix the inner wheel 201. The bottom of the cylindrical part is provided with a detection window 16, and the detection window 16 is located between the two rotating shaft support plates. Referring to FIG. 12, a supporting plate is provided inside the cylindrical part. An angle detection device 7 is provided on the top surface of the supporting plate, and a light sensor 13 and a light emitting device 14 are fixed on the bottom surface of the supporting plate.

The balance wheel 13 is further provided with a third sensor 10. The third sensor 10 is provided on the wheel surface or a wheel shaft of the balance wheel 3, and the third sensor 10 is configured to detect the position where the wheel surface of the balance wheel is in contact with a measured person.

The balance wheels 3 are located on both sides of the measuring wheel 2 and are connected with the measuring body 1, respectively. The axle direction of the balance wheel 3 is parallel to the axle of the measuring wheel 2. The balance wheels 3 are provided in pairs (the balance wheels 3 provided on the left and right sides of the measuring wheel 2 are referred to as a pair of balance wheels), which are not less than two pairs. During the measurement, the balance wheel 3 is in contact with the skin surface of the muscle high points on both sides of the spine.

The balance wheel 3 consists of an axle fixedly installed on the measuring body 1 and a rollable wheel surface. The height of the wheel surface cylinder is more than twice its diameter, and the height of the wheel surface is not less than 30mm. A pressure sensor or a strain sensor (the third sensor 10) is provided on the axle or the wheel surface of the balance wheel 3 to measure the contact pressure between the balance wheel 3 and the skin. When the contact pressure is zero, it can be judged that the measurement operation is deviated.

The control device is located in the measuring body 1. The control device comprises a control panel, a spatial coordinate trajectory data analyzing module, a data transmission module and a power management function module. The spine measuring device can be in communication connection with a computer, a tablet, a mobile terminal, an upper computer or other devices.

The present disclosure provides a spine measuring system, comprising an external host and the spine measurement device, wherein the external host is in communication connection with the spine measurement device.

The specific structure of the spine measuring device has been specifically stated above, which will not be described in detail here.

The present disclosure provides a measuring method for detecting a spine by using the spine measuring device, comprising:
acquiring pressure data of each coordinate position of the spine;
identifying a position of a seventh cervical spinous process based on the pressure data for positioning the detection of cervical vertebra.

The measuring method acquires pressure change data through a pressure sensor of a spine measuring device, accurately identifies and positions the seventh cervical vertebra, and finds the starting point of measurement accurately. Data acquisition also starts from the seventh cervical vertebra, thus ensuring the accuracy of subsequent data acquisition and analysis.

The step of "identifying a position of a seventh cervical spinous process based on the pressure data" specifically comprises;
acquiring pressure change data based on the pressure data of each coordinate position;
searching for target pressure change data which conforms to a preset seventh cervical pressure abrupt change range according to the pressure change data;
determining a corresponding measurement coordinate position according to the target pressure change data;
identifying the position of the seventh cervical spinous process based on the corresponding measurement coordinate position.

Combined with the above statement about the specific Embodiment 1 of the spine measuring device, the measuring method for detecting the spine is introduced as follows.

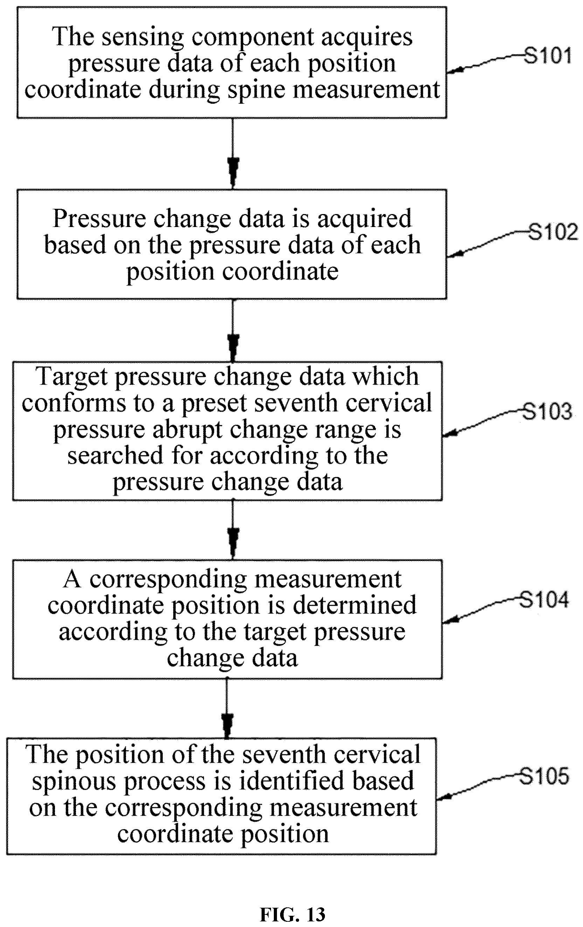

Referring to FIG. 13, the present disclosure provides a measuring method, comprising the following steps.

S101. The sensing component acquires pressure data of each coordinate position during spine measurement.

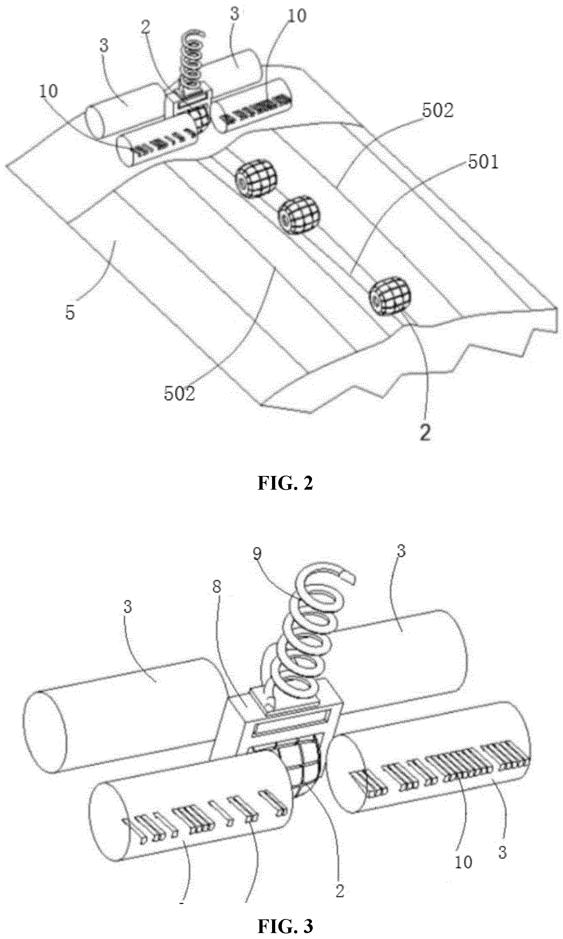

As shown in FIG. 2, an elastic element 9 (a telescopic spring) is installed on the mounting frame 8 on which the measuring wheel 2 is installed, so that the surface of the measuring wheel 2 can be kept in contact with the skin surface. The measuring wheel 2 is located in the middle of the balance wheels 3. The width of the measuring wheel 2 is smaller than the gap width between each pair of balance wheels 3. When the human spine is convex in the body surface area, the wheel surface of the measuring wheel 2 can be shrunk above the tangent plane of the wheel surface of the four balance wheels 3, and the protruding part of the human body is just located in the gap between each pair of balance wheels 3, so that the balance wheels 3 can continue to be in contact with the skin surface.

The sensing component comprises sensors installed on the measuring wheel 2, the balance wheel 3 and the mounting frame 8. The sensing component acquires pressure data of the measuring wheel 2 and the balance wheel 3 moving to each coordinate position during spine measurement.

S102. Pressure change data is acquired based on the pressure data of each coordinate position.

During the measurement of the sensing component, when the measuring wheel 2 moves on the uneven skin surface, the measuring wheel 2 will fluctuate with the unevenness, and the spring will compress accordingly.

S103. Target pressure change data which conforms to a preset seventh cervical pressure abrupt change range is searched for according to the pressure change data.

S104. A corresponding measurement coordinate position is determined according to the target pressure change data.

S105. The position of the seventh cervical spinous process is identified based on the corresponding measurement coordinate position.

Because the human body will be particularly convex in the seventh cervical spinous process, the pressure may abruptly change. By recording the abruptly changing pressure signal, the measurement position of the seventh cervical spinous process can be identified, which is used to further optimize the position of pathological points after measurement, so that the starting point of measurement can be found accurately. Data acquisition also starts from the seventh cervical vertebra, thus ensuring the accuracy of subsequent data acquisition and analysis.

After "determining a corresponding measurement coordinate position according to the target pressure change data", the step further comprises:
judging whether the spatial trajectory formed by the corresponding measurement coordinate position conforms to the trajectory abrupt change characteristic value standard of the seventh cervical vertebra;
if so, executing the process of identifying the position of the seventh cervical spinous process, that is, executing step S105.

In order to position the seventh cervical vertebra more accurate, on the basis of "searching for target pressure change data which conforms to a preset seventh cervical pressure abrupt change range", it is combined with the position change data. That is, when the device moves to the seventh cervical spinous process, the spatial trajectory will abruptly change due to the external characteristics of the seventh cervical vertebra. The seventh cervical vertebra can be positioned and identified by extracting the trajectory abrupt change characteristic value of the seventh cervical vertebra.

About "acquiring pressure data of each coordinate position during spine measurement by the sensing component", the specific explanation is as follows.

The pressure data of each coordinate position during spine measurement is acquired by the pressure sensor and the triaxial angle sensor.

Specifically, a pressure sensor 6 is installed on the end face of the telescopic spring on which the measuring wheel 2 is installed. During the measurement of the pressure sensor 6, when the measuring wheel 2 moves on the uneven skin surface, the measuring wheel 2 will fluctuate with the unevenness, and the spring will compress accordingly.

Three groups of capacitance sensors (first sensors 4) are installed side by side along the rolling direction on the wheel surface of the measuring wheel 2. Based on the capacitance data acquired by three groups of capacitance sensors and the acquired spatial curve data, the measured area of the human body where a spine detection handheld device is located can be judged.

When the measuring wheel rolls along the skin, the first sensors 4 are in contact with the skin in sequence. The current movement speed and rolling distance are calculated based on the contact interval time and the arc length of the capacitance sensor. Based on the current movement speed, the rolling distance and each coordinate position acquired by the triaxial angle sensor, the spatial curve data are acquired.

As shown in FIG. 2, a group of capacitance sensors are provided on either of the left, middle and right parts of the wheel surface of the measuring wheel 2. The sensing points of each group of capacitance sensors are uniformly distributed in the corresponding wheel surface area of the measuring wheel. Three groups of capacitance sensors form three circles of sensing points of sensors. The middle group of capacitance sensors are located on the central axis of the wheel surface of the measuring wheel, and the left and right groups of capacitance sensors are symmetrically distributed with the middle group of capacitance sensors as the axis.

When the middle group of capacitance sensors acquire capacitance data and the left and right groups of capacitance sensors do not acquire capacitance data, it is determined that the measured area of the human body where the spine detection handheld device is located is a cervical vertebra section. When the left and right groups of capacitance sensors acquire the capacitance data and the middle group of capacitance sensors do not acquire capacitance data, it is determined that the measured area of the human body where the spine detection handheld device is located is a lumbar vertebra section. When all three groups of capacitance sensors acquire capacitance data, it is determined that the measured area of the human body where the spine detection handheld device is located is the thoracic vertebra section.

Since the shape of the body surface area where the spine is located is convex in the neck area, only the middle group of the three groups of capacitance sensors can be in contact with the skin, while the left and right groups of capacitance sensors cannot be in contact with the skin. The body surface shape of the thoracic vertebra and lumbar vertebra gradually transits from an upper convex part to a lower concave part. At this time, the contact area between three groups of sensors and the skin gradually increases, in which at the beginning, only the middle group of capacitance sensors are in contact with the skin, and then the left and right groups of capacitance sensors are in contact with the skin, until the area of the middle group of capacitance sensors in contact with a very concave area decreases or even is not in contact with the very concave area. In this way, it can be judged that the spine measuring device is located in the measured area of the human body.

The present disclosure further provides a control device, comprising:
a sensing component, which is configured to acquire pressure data of each coordinate position during spine measurement;
an acquisition module, which is configured to acquire pressure change data based on the pressure data of each coordinate position;
a searching module, which is configured to search for target pressure change data which conforms to a preset seventh cervical pressure abrupt change range according to the pressure change data;
a determining module, which is configured to determine a corresponding measurement coordinate position according to the target pressure change data;
an identifying module, which is configured to identify the position of the seventh cervical spinous process based on the corresponding measurement coordinate position.

The control device further comprises a judging module, which is configured to:
judge whether the spatial trajectory formed by the corresponding measurement coordinate position conforms to the trajectory abrupt change characteristic value standard of the seventh cervical vertebra;
if so, execute the process of identifying the position of the seventh cervical spinous process.

The control device further comprises an analyzing and processing module, which is configured to calculate and acquire spatial curve data and spatial angle change data of a spine measuring point, and is configured to measure a cobb angle of scoliosis base on the spatial curve data and the spatial angle change data, so as to determine a scoliosis degree according to the cobb angle.

It can be understood that the same or similar parts in the above embodiments can refer to each other. The contents which are not explained in detail in some embodiments can refer to the same or similar contents in other embodiments.

In the description of the present disclosure, it should be noted that, unless otherwise stated, "a plurality of" means two or more; the orientational or positional relationships indicated by the terms such as "up", "down", "left", "right", "inside", "outside", "front end", "back end", "head" and "tail" are based on the orientational or positional relationships shown in the drawings only for the convenience of describing the present disclosure and simplifying the description, rather than indicate or imply that the referred devices or elements must have a specific orientation, be constructed and operated in a specific orientation, and therefore should not be construed as limiting the present disclosure. In addition, the terms such as "first", "second" and "third" are only used for the purpose of description, and cannot be understood as indicating or implying relative importance.

In the description of the present disclosure, it should also be noted that unless otherwise specified and defined expressly, the terms such as "mount", "link" and "connect" should be understood broadly, for example, it can be fixed connection, detachable connection or integral connection; or mechanical connection or electrical connection; or direct connection or indirect connection through an intermediate medium. For those skilled in the art, the specific meanings of the above terms in the present disclosure can be understood according to specific situations.

Any process or method description in the flow chart or otherwise described here can be understood as a module, segment or part of a code that comprises one or more executable instructions for implementing steps of specific logical functions or processes, and the scope of the preferred embodiments of the present disclosure comprises other implementations, in which functions can be performed out of the order shown or discussed, including in a substantially simultaneous manner or in a reverse order according to the functions involved, which should be understood by those skilled in the art to which the embodiments of the present disclosure belong.

It should be understood that various parts of the present disclosure can be implemented by hardware, software, firmware or the combination thereof. In the above embodiments, a plurality of steps or methods can be implemented by software or firmware stored in a memory and executed by a suitable instruction execution system. For example, if the steps or methods are implemented by hardware, as in another embodiment, the steps or methods can be implemented by any one of the following technologies known in the art or the combination thereof: a discrete logic circuit with a logic gate for realizing logic functions on data signals, an application specific integrated circuit with appropriate combined logic gates, a Programmable Gate Array (PGA), a Field Programmable Gate Array (FPGA), etc.

Those skilled in the art can understand that all or part of the steps involved in implementing the above embodiment method can be completed by instructing related hardware through a program, which can be stored in a computer-readable storage medium. When being executed, the program comprises one or a combination of the steps of the method embodiment.

In addition, each functional unit in each embodiment of the present disclosure may be integrated in one processing module, each unit may exist physically alone, or two or more units may be integrated in one module. The above integrated modules can be realized in the form of hardware or in the form of a software functional module. If the integrated module is implemented in the form of a software functional module and sold or used as an independent product, the integrated module can also be stored in a computer-readable storage medium.

The storage medium mentioned above can be a read-only memory, a magnetic disk or an optical disk, etc.

In the description of this specification, the description referring to the terms "one embodiment", "some embodiments", "examples", "specific examples" or "one example" means that the specific features, structures, materials or characteristics described in connection with this embodiment or example are included in at least one embodiment or example of the present disclosure. In this specification, the schematic expressions of the above terms do not necessarily refer to the same embodiment or example. Furthermore, the specific features, structures, materials or characteristics described may be combined in any one or more embodiments or examples in a suitable manner.

The above only describes the specific embodiments of the present disclosure, the invention being defined by the appended claims.

## Claims

1. A spine measuring device, comprising a measuring body (1), a measuring wheel (2), a balance wheel (3), a first detection device, a spatial coordinate acquisition device (7) and a control device,
wherein the measuring wheel (2) is provided at one end of the measuring body (1), at least four balance wheels (3) are symmetrically provided in rows at both sides of the measuring wheel (2) and are all connected with the measuring body (1), the first detection device and the spatial coordinate acquisition device (7) are both connected with the control device, the first detection device is configured to detect the movement of the measuring wheel (2), and the spatial coordinate acquisition device (7) is configured to detect a spatial angle value,
wherein the spine measuring device further comprises a mounting frame (8) and an elastic element (9), the mounting frame (8) is provided at one end of the measuring body (1), the measuring wheel (2) is installed on the mounting frame (8), the elastic element (9) is provided in the measuring body (1), one end of the elastic element (9) abuts against the measuring body (1), and the other end thereof is connected with the mounting frame (8), so that the measuring wheel (2) can move in the axial direction of the elastic element (9) and roll along the skin,
wherein the spatial coordinate acquisition device (7) is provided on the mounting frame (8), the spatial coordinate acquisition device (7) is opposite to the position of the measuring wheel (2), and the spatial coordinate acquisition device (7) comprises a triaxial angle sensor.

2. The spine measuring device according to claim 1, wherein the spine measuring device further comprises a pressure sensor (6) configured to measure the pressure on the measuring wheel (2), the pressure sensor (6) is connected with the control device, and the pressure sensor (6) is provided between the mounting frame (8) and the elastic element (9).

3. The spine measuring device according to claim 1 or 2, wherein the measuring wheel (2) is provided on a central axis of the measuring body (1); the first detection device comprises a plurality of first sensors (4), and the plurality of first sensors (4) are arranged along a rolling direction of a wheel surface of the measuring wheel (2).

4. The spine measuring device according to claim 3, wherein a radial section of any position of the measuring wheel (2) is circular, a diameter of the middle position of the measuring wheel (2) is larger than diameters of the left and right positions of the measuring wheel (2); the plurality of first sensors (4) are arranged in three groups along the rolling direction of the wheel surface of the measuring wheel (2), one group of the first sensors (4) is provided at the middle position of the measuring wheel (2), and the other two groups of the first sensors (4) are provided at both sides of the measuring wheel (2).

5. The spine measuring device according to claim 3, wherein an arc angle between sensing points of two adjacent of the first sensors (4) and the center of the measuring wheel is less than 72 degrees; alternatively, wherein the first sensors (4) are uniformly distributed on the wheel surface of the measuring wheel (2) to form three circles of sensors, and there are not less than 20 first sensors (4) in each circle.

6. The spine measuring device according to claim 1, wherein an optical sensor (13) and a light emitting device (14) are provided on the mounting frame (8) supporting the measuring wheel (2), the optical sensor (13) and the light emitting device (14) are provided above the measuring wheel (2), an outer surface of an outer wheel of the measuring wheel (2) is provided with reflective strips (15) with different light reflectivity at intervals along the rolling direction, and the intensity of the reflected light is received by the optical sensor for judging a rolling speed and a travel distance of the measuring wheel (2).

7. The spine measuring device according to claim 6, wherein the measuring wheel (2) comprises an inner wheel (201) and an outer wheel (202), the outer wheel (202) is sleeved on the inner wheel (201), the inner wheel (201) is fixed, and the outer wheel (202) rolls with respect to the inner wheel (201); more than two fourth sensors (12) are provided in a downward area of the outer surface of the inner wheel (201), the more than two fourth sensors (12) are provided along a circumferential direction of the inner wheel (201), the more than two fourth sensors (12) are connected with the control device, and the more than two fourth sensors (12) are configured to test a pressure change between the measuring wheel (2) and skin.

8. The spine measuring device according to claim 1, wherein the balance wheels (2) are further provided with a respective third sensor (10), the respective third sensor (10) is provided on the wheel surface or a wheel shaft of the respective balance wheel (3), and the respective third sensor (10) is configured to detect the position where the wheel surface of the respective balance wheel (3) is in contact with a measured person.

9. The spine measuring device according to claim 1, wherein either of a pair of balance wheels (3) provided oppositely is provided with a second sensor (11), the second sensor (11) is connected with the control device, and the second sensor (11) is configured to detect a moving distance of the balance wheels (3).

10. The spine measuring device according to claim 1, wherein a distance between the pair of balance wheels (3) provided oppositely is greater than a width of the measuring wheel (2); at least four balance wheels (3) rotate independently, and each of the balance wheels (3) has a cylindrical structure.

11. A spine measurement system, comprising an external host and the spine measuring device according to any of claims 1 to 9, wherein the external host is in communication connection with the spine measuring device.

12. A measuring method for detecting a spine by using the spine measuring device according to claim 2, comprising:
acquiring (S101) pressure data of each coordinate position of the spine by using the pressure sensor (6) of the spine measuring device;
identifying (S105) a position of a seventh cervical spinous process based on the pressure data for positioning the detection of cervical vertebra.

13. The measuring method according to claim 12, wherein identifying the position of the seventh cervical spinous process based on the pressure data specifically comprises:
acquiring (S102) pressure change data based on the pressure data of each coordinate position;
searching (S103) for target pressure change data which conforms to a preset seventh cervical pressure abrupt change range according to the pressure change data;
determining (S104) a corresponding measurement coordinate position according to the target pressure change data;
identifying (S105) the position of the seventh cervical spinous process based on the corresponding measurement coordinate position.

## Patentansprüche

1. Wirbelsäulenmessvorrichtung, umfassend einen Messkörper (1), ein Messrad (2), ein Unruhrad (3), eine erste Erfassungsvorrichtung, eine Raumkoordinaten-Erfassungsvorrichtung (7) und eine Steuervorrichtung,
wobei das Messrad (2) an einem Ende des Messkörpers (1) vorgesehen ist, mindestens vier Unruhräder (3) symmetrisch in Reihen an beiden Seiten des Messrads (2) vorgesehen und alle mit dem Messkörper (1) verbunden sind, die erste Erfassungsvorrichtung und die Raumkoordinaten-Erfassungsvorrichtung (7) beide mit der Steuervorrichtung verbunden sind, die erste Erfassungsvorrichtung dazu konfiguriert ist, die Bewegung des Messrads (2) zu erfassen, und die Raumkoordinaten-Erfassungsvorrichtung (7) dazu konfiguriert ist, einen Raumwinkelwert zu erfassen,
wobei die Wirbelsäulenmessvorrichtung ferner eine Montagehalterung (8) und ein elastisches Element (9) umfasst, die Montagehalterung (8) an einem Ende des Messkörpers (1) vorgesehen ist, das Messrad (2) an der Montagehalterung (8) montiert ist, das elastische Element (9) in dem Messkörper (1) vorgesehen ist, ein Ende des elastischen Elements (9) gegen den Messkörper (1) drückt und das andere Ende davon mit der Montagehalterung (8) verbunden ist, so dass sich das Messrad (2) in der axialen Richtung des elastischen Elements (9) bewegen und auf der Haut abrollen kann,
wobei die Raumkoordinaten-Erfassungsvorrichtung (7) an der Montagehalterung (8) vorgesehen ist, die Raumkoordinaten-Erfassungsvorrichtung (7) der Position des Messrads (2) gegenüberliegt und die Raumkoordinaten-Erfassungsvorrichtung (7) einen dreiachsigen Winkelsensor umfasst.

2. Wirbelsäulenmessvorrichtung nach Anspruch 1, wobei die Wirbelsäulenmessvorrichtung ferner einen Drucksensor (6) umfasst, der zum Messen des Drucks auf das Messrad (2) konfiguriert ist, der Drucksensor (6) mit der Steuervorrichtung verbunden ist und der Drucksensor (6) zwischen der Montagehalterung (8) und dem elastischen Element (9) vorgesehen ist.

3. Wirbelsäulenmessvorrichtung nach Anspruch 1 oder 2, wobei das Messrad (2) auf einer Mittelachse des Messkörpers (1) vorgesehen ist; die erste Erfassungsvorrichtung mehrere erste Sensoren (4) umfasst, und die mehreren ersten Sensoren (4) entlang einer Rollrichtung einer Radoberfläche des Messrads (2) angeordnet sind.

4. Wirbelsäulenmessvorrichtung nach Anspruch 3, wobei der radiale Querschnitt an jeder Position des Messrads (2) kreisförmig ausgebildet ist, ein Durchmesser der mittleren Position des Messrads (2) größer ist als die Durchmesser der linken und rechten Positionen des Messrads (2); die mehreren ersten Sensoren (4) in drei Gruppen entlang der Rollrichtung der Radoberfläche des Messrads (2) angeordnet sind, eine Gruppe der ersten Sensoren (4) an der mittleren Position des Messrads (2) vorgesehen ist und die anderen zwei Gruppen der ersten Sensoren (4) an beiden Seiten des Messrads (2) vorgesehen sind.

5. Wirbelsäulenmessvorrichtung nach Anspruch 3, wobei ein Bogenwinkel zwischen den Messpunkten zweier benachbarter erster Sensoren (4) und der Mitte des Messrads weniger als 72 Grad beträgt; alternativ, wobei die ersten Sensoren (4) gleichmäßig auf der Radoberfläche des Messrads (2) verteilt sind, um drei Sensorkreise zu bilden, und nicht weniger als 20 erste Sensoren (4) in jedem Kreis vorhanden sind.

6. Wirbelsäulenmessvorrichtung nach Anspruch 1, wobei ein optischer Sensor (13) und eine Lichtemissionsvorrichtung (14) an der Montagehalterung (8) vorgesehen sind, die das Messrad (2) stützt, der optische Sensor (13) und die Lichtemissionsvorrichtung (14) über dem Messrad (2) vorgesehen sind, eine Außenfläche eines äußeren Rads des Messrads (2) mit reflektierenden Streifen (15) mit unterschiedlichem Lichtreflexionsvermögen in Intervallen entlang der Rollrichtung versehen ist, und die Intensität des reflektierten Lichts durch den optischen Sensor empfangen wird, um eine Rollgeschwindigkeit und eine Fahrstrecke des Messrads (2) zu beurteilen.

7. Wirbelsäulenmessvorrichtung nach Anspruch 6, wobei das Messrad (2) ein inneres Rad (201) und ein äußeres Rad (202) umfasst, das innere Rad (201) von dem äußeren Rad (202) umgeben ist, das innere Rad (201) fixiert ist und das äußere Rad (202) relativ zu dem inneren Rad (201) rollt; mehr als zwei vierte Sensoren (12) in einem Abwärtsbereich der Außenfläche des inneren Rads (201) vorgesehen sind, die mehr als zwei vierten Sensoren (12) entlang einer Umfangsrichtung des inneren Rads (201) vorgesehen sind, die mehr als zwei vierten Sensoren (12) mit der Steuervorrichtung verbunden sind, und die mehr als zwei vierten Sensoren (12) dazu konfiguriert sind, eine Druckänderung zwischen dem Messrad (2) und der Haut zu prüfen.

8. Wirbelsäulenmessvorrichtung nach Anspruch 1, wobei die Unruhräder (2) ferner mit einem jeweiligen dritten Sensor (10) versehen sind, der jeweilige dritte Sensor (10) auf der Radoberfläche oder einer Radwelle des jeweiligen Unruhrads (3) vorgesehen ist, und der jeweilige dritte Sensor (10) dazu konfiguriert ist, die Position zu erfassen, an der die Radoberfläche des jeweiligen Unruhrads (3) in Kontakt mit einer gemessenen Person ist.

9. Wirbelsäulenmessvorrichtung nach Anspruch 1, wobei eines aus einem Paar von gegenüberliegenden Unruhrädern (3) mit einem zweiten Sensor (11) versehen ist, der zweite Sensor (11) mit der Steuervorrichtung verbunden ist und der zweite Sensor (11) dazu konfiguriert ist, einen Bewegungsabstand der Unruhräder (3) zu erfassen.

10. Wirbelsäulenmessvorrichtung nach Anspruch 1, wobei der Abstand zwischen dem Paar von gegenüberliegenden Unruhrädern (3) größer ist als die Breite des Messrads (2); mindestens vier Unruhräder (3) sich unabhängig voneinander drehen und jedes der Unruhräder (3) eine zylindrische Struktur aufweist.

11. Wirbelsäulenmesssystem, umfassend einen externen Host und die Wirbelsäulenmessvorrichtung nach einem der Ansprüche 1 bis 9, wobei der externe Host in Kommunikationsverbindung mit der Wirbelsäulenmessvorrichtung steht.

12. Messverfahren zum Erfassen einer Wirbelsäule unter Verwendung der Wirbelsäulenmessvorrichtung nach Anspruch 2, umfassend:
Erfassen (S101) von Druckdaten für jede Koordinatenposition der Wirbelsäule unter Verwendung des Drucksensors (6) der Wirbelsäulenmessvorrichtung;
Identifizieren (S105) einer Position eines siebten zervikalen Dornfortsatzes basierend auf den Druckdaten zum Positionieren von Halswirbelerfassung.

13. Messverfahren nach Anspruch 12, wobei das Identifizieren der Position des siebten zervikalen Dornfortsatzes basierend auf den Druckdaten insbesondere Folgendes umfasst:
Erfassen (S102) von Druckänderungsdaten basierend auf den Druckdaten jeder Koordinatenposition;
Suchen (S103) nach Zieldruckänderungsdaten, die mit einem voreingestellten Bereich der abrupten Änderung des siebten zervikalen Drucks übereinstimmen, gemäß den Druckänderungsdaten;
Bestimmen (S104) einer entsprechenden Messkoordinatenposition gemäß den Zieldruckänderungsdaten;
Identifizieren (S105) der Position des siebten zervikalen Dornfortsatzes basierend auf der entsprechenden Messkoordinatenposition.

## Revendications

1. Dispositif de mesure de colonne vertébrale, comprenant un corps de mesure (1), une roue de mesure (2), une roue d'équilibre (3), un premier dispositif de détection, un dispositif d'acquisition de coordonnées spatiales (7) et un dispositif de commande,
dans lequel la roue de mesure (2) est disposée au niveau d'une extrémité du corps de mesure (1), au moins quatre roues d'équilibre (3) sont disposées symétriquement en rangées au niveau de deux côtés de la roue de mesure (2) et sont toutes reliées au corps de mesure (1), le premier dispositif de détection et le dispositif d'acquisition de coordonnées spatiales (7) sont tous deux reliés au dispositif de commande, le premier dispositif de détection est configuré pour détecter le déplacement de la roue de mesure (2), et le dispositif d'acquisition de coordonnées spatiales (7) est configuré pour détecter une valeur d'angle spatial,
dans lequel le dispositif de mesure de colonne vertébrale comprend en outre un cadre de montage (8) et un élément élastique (9), le cadre de montage (8) est disposé au niveau d'une extrémité du corps de mesure (1), la roue de mesure (2) est montée sur le cadre de montage (8), l'élément élastique (9) est disposé dans le corps de mesure (1), une extrémité de l'élément élastique (9) vient en butée contre le corps de mesure (1), et l'autre extrémité de celui-ci est reliée au cadre de montage (8), de sorte que la roue de mesure (2) peut se déplacer dans la direction axiale de l'élément élastique (9) et rouler le long de la peau,
dans lequel le dispositif d'acquisition de coordonnées spatiales (7) est disposé sur le cadre de montage (8), le dispositif d'acquisition de coordonnées spatiales (7) est opposé à la position de la roue de mesure (2) et le dispositif d'acquisition de coordonnées spatiales (7) comprend un capteur d'angle triaxial.

2. Dispositif de mesure de colonne vertébrale selon la revendication 1, dans lequel le dispositif de mesure de colonne vertébrale comprend en outre un capteur de pression (6) configuré pour mesurer la pression sur la roue de mesure (2), le capteur de pression (6) est relié au dispositif de commande, et le capteur de pression (6) est disposé entre le cadre de montage (8) et l'élément élastique (9).

3. Dispositif de mesure de colonne vertébrale selon la revendication 1 ou 2, dans lequel la roue de mesure (2) est disposée sur un axe central du corps de mesure (1) ; le premier dispositif de détection comprend une pluralité de premiers capteurs (4), et la pluralité de premiers capteurs (4) sont disposés le long d'une direction de roulement d'une surface de roue de la roue de mesure (2).

4. Dispositif de mesure de colonne vertébrale selon la revendication 3, dans lequel une section radiale d'une quelconque position de la roue de mesure (2) est circulaire, un diamètre de la position centrale de la roue de mesure (2) est plus grand que des diamètres des positions gauche et droite de la roue de mesure (2) ; la pluralité de premiers capteurs (4) sont disposés en trois groupes le long de la direction de roulement de la surface de roue de la roue de mesure (2), un groupe des premiers capteurs (4) est disposé au niveau de la position centrale de la roue de mesure (2), et les deux autres groupes des premiers capteurs (4) sont disposés au niveau de deux côtés de la roue de mesure (2).

5. Dispositif de mesure de colonne vertébrale selon la revendication 3, dans lequel un angle d'arc entre des points de détection de deux capteurs adjacents des premiers capteurs (4) et le centre de la roue de mesure est inférieur à 72 degrés ; alternativement, dans lequel les premiers capteurs (4) sont uniformément répartis sur la surface de roue de la roue de mesure (2) afin de former trois cercles de capteurs, et il n'y a pas moins de 20 premiers capteurs (4) dans chaque cercle.

6. Dispositif de mesure de colonne vertébrale selon la revendication 1, dans lequel un capteur optique (13) et un dispositif d'émission de lumière (14) sont disposés sur le cadre de montage (8) soutenant la roue de mesure (2), le capteur optique (13) et le dispositif d'émission de lumière (14) sont disposés au-dessus de la roue de mesure (2), une surface externe d'une roue externe de la roue de mesure (2) est pourvue de bandes réfléchissantes (15) ayant une réflectivité lumineuse différente à intervalles le long de la direction de roulement, et l'intensité de la lumière réfléchie est reçue par le capteur optique afin d'évaluer une vitesse de roulement et une distance de déplacement de la roue de mesure (2).

7. Dispositif de mesure de colonne vertébrale selon la revendication 6, dans lequel la roue de mesure (2) comprend une roue interne (201) et une roue externe (202), la roue externe (202) est manchonnée sur la roue interne (201), la roue interne (201) est fixe, et la roue externe (202) roule par rapport à la roue interne (201) ; plus de deux quatrièmes capteurs (12) sont disposés dans une zone descendante de la surface externe de la roue interne (201), les plus de deux quatrièmes capteurs (12) sont disposés le long d'une direction circonférentielle de la roue interne (201), les plus de deux quatrièmes capteurs (12) sont reliés au dispositif de commande, et les plus de deux quatrièmes capteurs (12) sont configurés pour tester un changement de pression entre la roue de mesure (2) et la peau.

8. Dispositif de mesure de colonne vertébrale selon la revendication 1, dans lequel les roues d'équilibre (2) sont en outre pourvues d'un troisième capteur (10) respectif, le troisième capteur (10) respectif est disposé sur la surface de roue de la roue ou sur un arbre de roue de la roue d'équilibre (3), et le troisième capteur (10) respectif est configuré pour détecter la position où la surface de roue de la roue d'équilibre (3) respectif est en contact avec une personne mesurée.

9. Dispositif de mesure de colonne vertébrale selon la revendication 1, dans lequel l'une ou l'autre d'une paire de roues d'équilibre (3) disposées de manière opposée est pourvu d'un second capteur (11), le second capteur (11) est relié au dispositif de commande, et le second capteur (11) est configuré pour détecter une distance de déplacement des roues d'équilibre (3).

10. Dispositif de mesure de colonne vertébrale selon la revendication 1, dans lequel une distance entre la paire de roues d'équilibre (3) disposées de manière opposée est supérieure à une largeur de la roue de mesure (2) ; au moins quatre roues d'équilibre (3) tournent indépendamment, et chacune des roues d'équilibre (3) a une structure cylindrique.

11. Système de mesure de colonne vertébrale comprenant un hôte externe et le dispositif de mesure de colonne vertébrale selon l'une quelconque des revendications 1 à 9, dans lequel l'hôte externe est en communication avec le dispositif de mesure de colonne vertébrale.

12. Procédé de mesure destinée à détecter une colonne vertébrale à l'aide du dispositif de mesure de colonne vertébrale selon la revendication 2, comprenant :
l'acquisition (S101) de données de pression de chaque position de coordonnée de la colonne vertébrale à l'aide du capteur de pression (6) du dispositif de mesure de colonne vertébrale ;
l'identification (S105) d'une position d'une septième apophyse épineuse cervicale sur la base des données de pression pour le positionnement de la détection de la vertèbre cervicale.

13. Procédé de mesure selon la revendication 12, dans lequel l'identification de la position de la septième apophyse épineuse cervicale sur la base des données de pression comprend spécifiquement :
l'acquisition (S102) de données de changement de pression sur la base des données de pression de chaque position de coordonnée ;
la recherche (S103) de données de changement de pression cibles qui sont conformes à une plage prédéfinie de changement brusque de septième apophyse cervicale en fonction des données de changement de pression ;
la détermination (S104) d'une position de coordonnée de mesure correspondante en fonction des données de changement de pression cibles ;
l'identification (S105) de la position de la septième apophyse épineuse cervicale sur la base de la position de coordonnée de mesure correspondante.
